# EUROPEAN PATENT SPECIFICATION

(11) **EP 2 601 522 B1**
(45) Date of publication and mention of the grant of the patent: **25.06.2014**
(21) Application number: 11741678.4
(22) Date of filing: 03.08.2011
(51) Int. Cl.: G01N 33/50, G01N 33/68

(54) **Method for diagnosing pre-eclampsia**
Methode zur Diagnose von Präeklampsie
Méthode de diagnostic de la prééclampsie

(30) Priority: 04.08.2010 GB 201013151
(43) Date of publication of application: 12.06.2013
(73) Proprietor: Isis Innovation Limited, Summertown, Oxford OX2 7SG (GB)
(72) Inventor: SARGENT, Ian, Kidlington Oxon OX5 1TF (GB); SOUTHCOMBE, Jennifer, Oxford OX3 0HP (GB); GRANNE, Ingrid, Headington Hill Oxford OX3 0BT (GB)
(74) Representative: Roques, Sarah Elizabeth
(86) International application number: PCT/GB2011/001167
(87) International publication number: WO 2012/017203

(56) References cited:
- WO-A1-01/42506
- WO-A1-2004/065629
- WO-A1-2005/031364
- WO-A2-2007/130627
- US-A1- 2004 146 501
- US-A1- 2005 170 444
- ANGELA&EMSP13;M. BORZYCHOWSKI ET AL: "Changes in systemic type 1 and type 2 immunity in normal pregnancy and pre-eclampsia may be mediated by natural killer cells", EUROPEAN JOURNAL OF IMMUNOLOGY, vol. 35, no. 10, 1 October 2005 (2005-10-01), pages 3054-3063, XP55010199, ISSN: 0014-2980, DOI: 10.1002/eji.200425929
- SARGENT ET AL: "NK cells and pre-eclampsia", JOURNAL OF REPRODUCTIVE IMMUNOLOGY, ELSEVIER SCIENCE IRELAND LTD, IE, vol. 76, no. 1-2, 15 November 2007 (2007-11-15), pages 40-44, XP022345529, ISSN: 0165-0378, DOI: 10.1016/J.JRI.2007.03.009
- GERMAIN SARAH J ET AL: "Systemic inflammatory priming in normal pregnancy and preeclampsia: The role of circulating syncytiotrophoblast microparticles", JOURNAL OF IMMUNOLOGY, AMERICAN ASSOCIATION OF IMMUNOLOGISTS, US, vol. 178, no. 9, 1 May 2007 (2007-05-01), pages 5949-5956, XP002608053, ISSN: 0022-1767
- DINARELLO C A: "An IL-1 Family Member Requires Caspase-1 Processing and Signals through the ST2 Receptor", IMMUNITY, CELL PRESS, US, vol. 23, 1 November 2005 (2005-11-01), pages 461-462, XP003021549, ISSN: 1074-7613, DOI: 10.1016/J.IMMUNI.2005.10.004
- INGRID GRANNE ET AL: "ST2 and IL-33 in Pregnancy and Pre-Eclampsia", PLOS ONE, vol. 6, no. 9, 1 January 2011 (2011-01-01) , pages E24463-E24463, XP55010209, ISSN: 1932-6203, DOI: 10.1371/journal.pone.0024463

## Description

### Field of the invention

The invention relates to a method of diagnosing pre-eclampsia or assessing the risk of a pregnant female developing pre-eclampsia.

### Background of the invention

Pre-eclampsia is a systemic syndrome of pregnancy that can be life-threatening to both mothers and fetuses. Pre-eclampsia affects approximately between 3-5% of pregnant women and is a leading cause of maternal mortality in the developing world.

During normal placental development, trophoblasts invade the maternal endometrium. The trophoblasts penetrate the endometrial blood vessels to form intertrophoblastic sinuses. These sinuses are fed by spiral arteries and fill with maternal blood. The spiral arteries dilatate and blood flow to the placenta increases.

Pre-eclampsia is caused by the insufficient invasion of trophoblasts into the developing placenta. This results in a restricted blood flow in the placenta. The oxidative stress resulting from the reduced placental blood flow provokes a systemic inflammatory response in the mother. Levels of circulating cytokines and complement proteins are increased in pregnant women suffering from pre-eclampsia. This results in endothelial cell dysfunction, leading to increased clotting, vasoconstriction and hypertension. The maternal vascular endothelium becomes damaged and its permeability increases, leading to tissue oedema, which can be particularly harmful if fluid accumulates in the brain. An increase in permeability in the renal vasculature results in protein that would normally be reabsorbed in the kidneys being excreted in the urine. Proteinuria and hypertension are commonly used to confirm a diagnosis of pre-eclampsia.

There is currently no screening method for pre-eclampsia that is accurate enough for clinical use. This is due to the absence of a sufficiently specific and sensitive biomarker to predict early in pregnancy which women are most at risk of pre-eclampsia. Although biomarkers of pre-eclampsia are known and used for research purposes, most of these are markers of endothelial cell dysfunction and do not increase significantly until the clinical disease is already present.

Pre-eclampsia is currently diagnosed based on the detection of two symptoms: (i) acute hypertension and (ii) abnormal proteinuria developing after 20 weeks gestation in a previously normotensive woman (Davey DA et al. Am J Obstet Gynecol 1988; 158: 892-8). Therefore, currently it is only possible to make a diagnosis of pre-eclampsia after a pregnant female presents with symptoms of the disease.

Documents WO 01/42506 and US 2004/146501 each document taken alone, disclose: A method of assessing/monitoring/detecting/prognosing/predicting the development or progression of (therefore assessing the risk of developing) T1R-like ligand II-related disorders/diseases such as e.g. pre-eclampsia comprising determining the level of soluble or mature forms of T1R-like ligand II polypeptide. The expression level of T1/ST2 (or sST2) and the binding activity T1R-like ligand II polypeptide to said receptor are as well measured in a sample.

The development of a reliable method of assessing risk of pre-eclampsia would enable closer pre-natal monitoring and intervention when necessary. Therefore, a reliable biological or clinical marker to assess the risk of pre-eclampsia as early as possible, preferably before the onset of clinical pathology is needed.

ST2 is a member of the IL-1 receptor family and is expressed in both membrane-bound (ST2L) and soluble (sST2) forms. IL-33 has recently been identified as a ligand for ST2L, with sST2 acting as a decoy receptor. IL-33 is a cytokine that promotes allergic Th2-type inflammatory responses (antibody mediated) such as asthma and septic shock. IL-33 interacts with cells via ST2L expressed on the surface of Th2 cells. sST2 levels in pregnant females have not previously been investigated.

### Summary of the invention

The present inventors have shown for the first time that levels of sST2 are significantly raised in pregnant females suffering from pre-eclampsia. Surprisingly, an increase in the level of sST2 in pregnant females can be detected before the onset of clinical symptoms.

According to the invention there is thus provided a method of assessing risk of developing pre-eclampsia comprising determining the level of sST2 in a sample from a pregnant female and thereby assessing risk of pre-eclampsia.

The invention further provides a test kit for use in a method for assessing risk of developing pre-eclampsia, which test kit comprises an agent for the detection of sST2 in a sample from a pregnant female and instructions for carrying out a method of assessing risk of developing pre-eclampsia.

### Description of the Figures

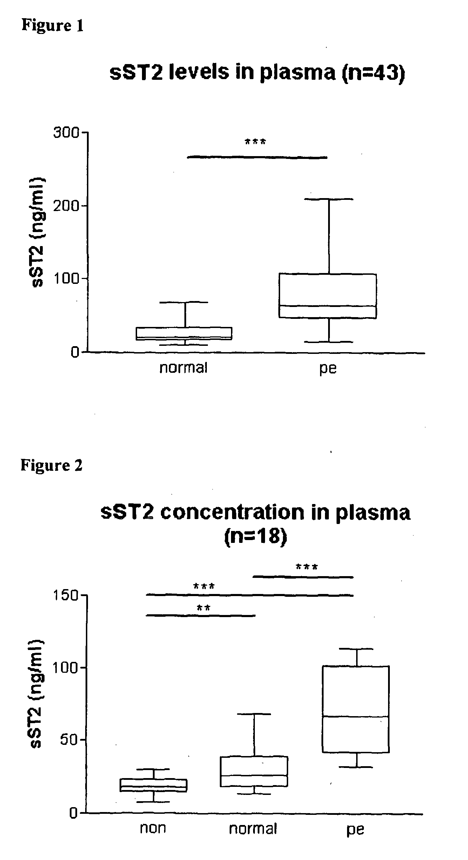

Figure 1 shows the concentration of circulating sST2 (ng/ml) in blood samples taken from 43 women having normal pregnancies and from 43 pregnant women suffering from pre-eclampsia matched for age, parity and gestational age. The box and whisker plots show the median, 25^{th} and 75^{th} percentile and the minimum and maximum of all the data. The circulating concentration of sST2 was significantly increased in the women suffering from pre-eclampsia compared to that in women having normal pregnancies (*** p≤ 0.001).
Figure 2 shows the concentration of circulating sST2 (ng/ml) in blood samples taken from 18 age and parity matched trios of non-pregnant women, women having normal pregnancies and pregnant women suffering from pre-eclampsia. The box and whisker plots show the median, 25^{th} and 75^{th} percentile and the minimum and maximum of all the data. Circulating sST2 was detectable in non-pregnant women. The levels were significantly increased in normal pregnant women (**p<0.01) and further increased in the women suffering from pre-eclampsia compared to that in women having normal pregnancies (*** p≤ 0.001) and non-pregnant women (**p≤ 0.01).

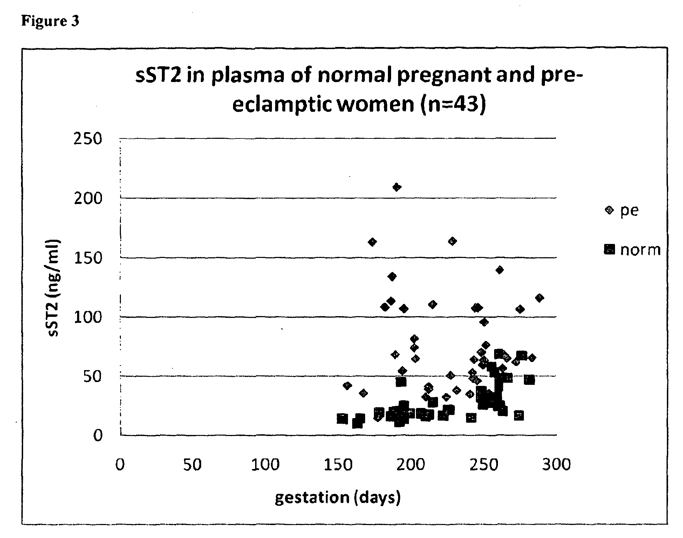

Figure 3 shows a scatter-plot illustrating the relationship between circulating sST2 concentrations (ng/ml) and the number of days gestation for 43 women having normal pregnancies (■) and from 43 pregnant women suffering from pre-eclampsia (◆). In normal pregnancy, circulating sST2 increases towards term. In pre-eclampsia, high circulating sST2 levels were detected from 150 days gestation onwards.

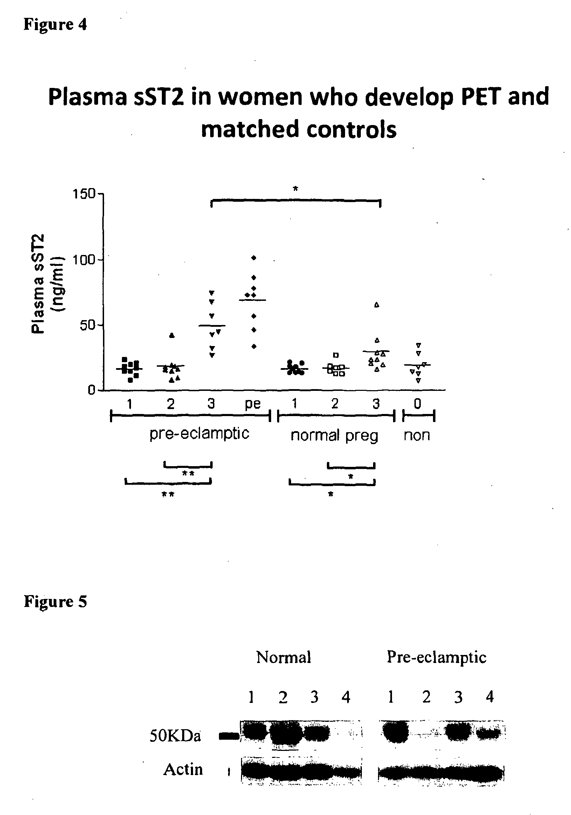

Figure 4 shows the levels of sST2 in the first, second and third trimesters of pregnancy in 7 women who did and 7 who did not develop pre-eclampsia. The levels of sST2 are significantly elevated in the third trimester before the onset of the disease, compared to matched normal pregnant women of the same gestation.
Figure 5 shows that sST2 is expressed in both normal and pre-eclampsia placentas.

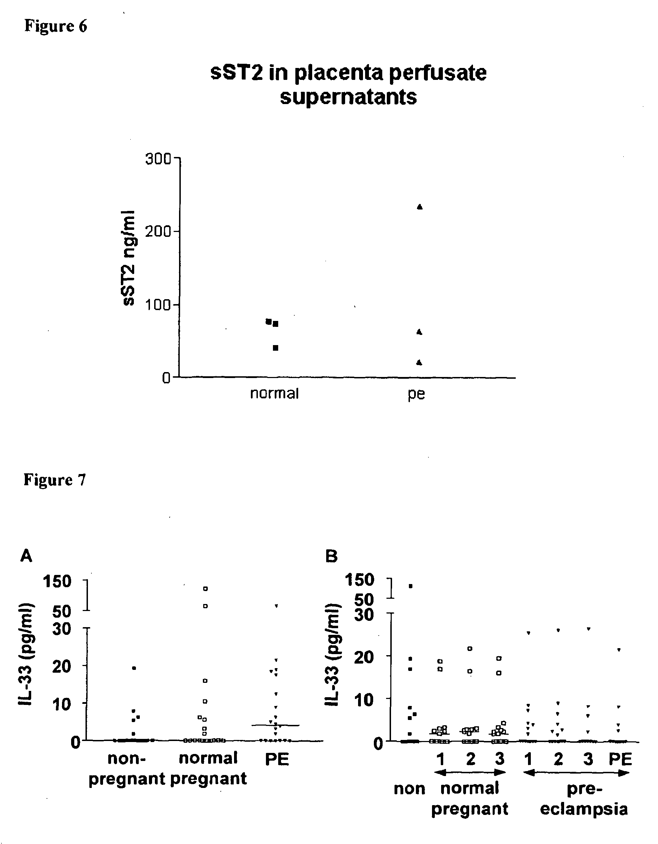

Figure 6 demonstrates that sST2 can be isolated from the placenta as it can be detected in placental perfusates prepared *ex vivo.*
Figure 7 - Circulating IL-33 in pregnancy and pre-eclampsia: A) IL-33 was detected by ELISA in the serum from women with a diagnosis of pre-eclampsia, matched controls who had a normal pregnancy and matched non-pregnant women (n=20). No significant differences in IL-33 were identified. Bars represent median values. B) IL-33 was detected by ELISA in samples taken throughout pregnancy in women who developed pre-eclampsia and matched controls who had an uncomplicated pregnancy (n=15). Blood samples were taken in each trimester of pregnancy (1, 2, 3) and at the diagnosis of pre-eclampsia (PE), and compared to non-pregnant matched women. There were no significant differences throughout pregnancy or between normal and pre-eclampsia pregnancies. Bars represent median values.

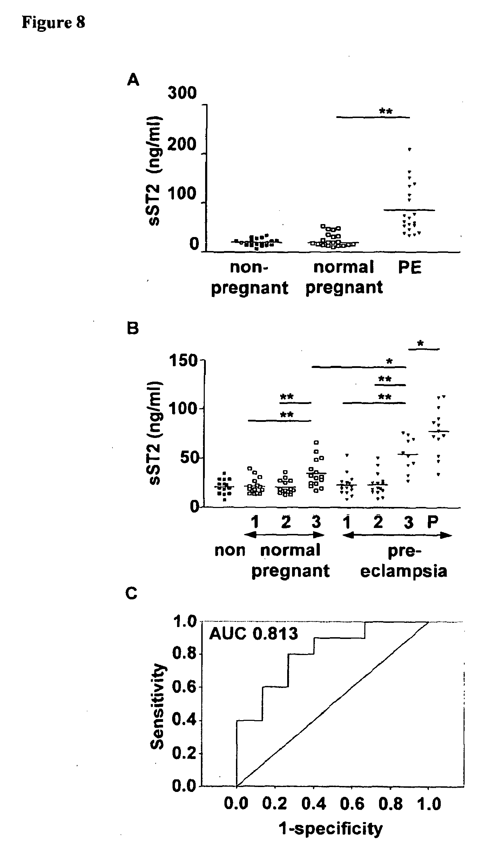

Figure 8 - Circulating sST2 in pregnancy and pre-eclampsia: A) sST2 was detected by ELISA in the plasma from women with a diagnosis of pre-eclampsia, matched controls who had a normal pregnancy and matched non-pregnant women (n=20). sST2 was significantly raised in pre-eclampsia compared to normal pregnancy (p< 0.001). B) sST2 was detected by ELISA in samples taken throughout pregnancy in women who developed pre-eclampsia and matched controls who had an uncomplicated pregnancy (n=15). Blood samples were taken in each trimester of pregnancy (1, 2, 3) and at the diagnosis of pre-eclampsia (PE), and compared to non-pregnant matched women. There is a significant increase in plasma sST2 in the third trimester of normal pregnancy compared to the first or second trimester (p<0.001), and compared to the non-pregnant group (p<0.001). sST2 was significantly raised in pre-eclampsia compared to normal pregnancy during the third trimester (p< 0.01), prior to the onset of clinical symptoms. Levels further increased when pre-eclampsia symptoms presented (p<0.05). C) ROC curve analysis indicates sST2 levels in the third trimester sample (n=10) are likely to predict pre-eclampsia (AUC=0.813). Bars represent mean values, * = p<0.05,** = p<0.01, *** = p<0.001.

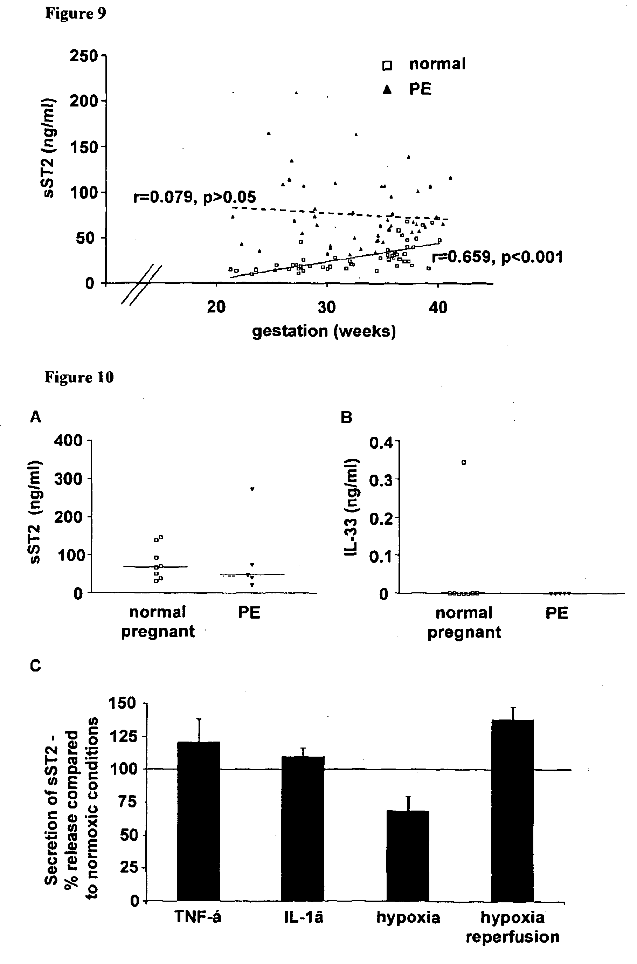

Figure 9 - Comparison of sST2 levels to gestational age: A) sST2 was detected by ELISA in plasma from women with a diagnosis of pre-eclampsia and matched controls who had a normal pregnancy (n=50). In normal pregnancy sST2 levels increase towards term. In pre-eclampsia sST2 levels are significantly increased compared to controls (p< 0.001). Differences in sST2 levels in normal and pre-eclampsia pregnancies were more apparent in early onset disease.
Figure 10 - The placenta releases sST2: sST2 (A) and IL-33 (B) were detected by ELISA in maternal side perfusate from an *ex vivo* of placental perfusion model. Normal (n=8) and pre-eclampsia (PE) (n=5) placentas were perfused. sST2 could be identified in media from all of the normal and pre-eclampsia placental perfusions, IL-33 could be identified in one sample from a normal pregnancy. Bars represent median values. C) Placental explants release sST2, secretion was increased by treatment with TNFα and IL-1β (100ng/ml) and hypoxia-reperfusion injury, and was decreased under hypoxic conditions (n=3).

### Detailed description of the invention

### Diagnosis

The present invention relates to a method of assessing risk of developing pre-eclampsia comprising determining the level of sST2 in a sample from a pregnant female and thereby assessing risk of pre-eclampsia. The invention therefore relates to the diagnosis of pre-eclampsia.

The sequence of ST2 is publicly available (for example as NCBI accession no. BAA02233) and is reproduced below as SEQ ID NO.1
SEQ ID NO: 1

The present inventors have for the first time investigated the level of sST2 in pregnant females. The inventors have demonstrated that sST2 levels are elevated in pregnant females suffering from pre-eclampsia relative to those pregnant females who do not have pre-eclampsia.

The method of the invention can be used to assess the risk of a pregnant female developing pre-eclampsia before the pregnant female presents with any symptoms. The method of the invention may be used to stratify pregnant females into high or low risk pregnancy. The method of the invention can also be used to diagnose or aid in the diagnosis of pre-eclampsia. Antenatal care may then be tailored accordingly. This would allow females at high risk to be closely monitored during pregnancy and would potentially save money in reducing hospitalisation of low risk pregnancies.

In one embodiment, the method of the invention may be carried out on pregnant females who have pre-eclampsia but have not been diagnosed with pre-eclampsia. The method may also be carried out on pregnant females who display preliminary symptoms of pre-eclampsia. In a preferred embodiment the pregnant female does not have pre-eclampsia or displays no symptoms of pre-eclampsia.

The test can be carried out on a female who shows partial signs without symptoms, of pre-eclampsia, for example new hypertension, which are non-specific and may or may not be associated with incipient pre-eclampsia. The pregnant female may present with new hypertension and abnormal proteinuria developing after 20 weeks gestation. Typically, the onset of pre-eclampsia is insiduous and occurs over three to four weeks. Typically, the method is carried out on a female during the third trimester of pregnancy, from 24 weeks gestation.

Other symptoms of pre-eclampsia that may be present include acute hypertension; abnormal proteinuria; pulmonary oedema; cyanosis; acute liver injury; acute renal failure; visual or cerebral disturbances; seizures; pain in the epigastric area or right upper quadrant; thrombocytopenia; hemolysis, intrauterine-growth restriction; placental abruption or oliguria.

The pregnant female may be suspected of being at risk of pre-eclampsia because of the presence of one or more risk factors. Risk factors for pre-eclampsia include:
- advanced maternal age or teenage mother;
- the pregnant female being nulliparous;
- a short interval between first coitus and conception with a new partner, regardless of previous pregnancy history;
- presentation with pre-eclampsia in a previous pregnancy;
- a history of pre-eclampsia in a first-degree relative;
- chronic hypertension;
- diabetes mellitus;
- renal disease;
- obesity;
- hypercoaguable states;
- multiple pregnancy; and
- certain autoimmune conditions, particularly antiphospholipid antibody syndrome.

The pregnant female being tested is typically a human.

The present invention involves measuring sST2 in a pregnant female. Typically the level or concentration of sST2 is measured. According to the present invention, an increased level or concentration of sST2 compared with the baseline level or concentration indicates that the pregnant female is at risk of developing pre-eclampsia. The baseline level is typically the level of sST2 in a pregnant female who presents no symptoms of pre-eclampsia throughout the entire pregnancy. According to the present invention, an increased level or concentration of sST2 compared with the level or concentration of sST2 in a normal pregnancy indicates that the pregnant female is at risk of developing pre-eclampsia. The normal pregnant female is typically at an equivalent stage of pregnancy to the pregnant female at risk of developing pre-eclampsia.

The circulating blood level of sST2 concentration in a normal non-pregnant female is typically low. For example, the inventors have detected a mean circulating blood level of 18.77 ng/ml sST2 in a group of non-pregnant females (n=18). Generally the circulating blood sST2 level in a normal pregnant female is higher than that in a normal non-pregnant female and increases during the gestation period. The circulating blood level of sST2 in a pregnant female suffering from pre-eclampsia is higher than that of a normal pregnant female.

For example the inventors have shown that, when the circulating blood level of sST2 is measured by determining the concentration of sST2 in a sample obtained from a pregnant female, the mean sST2 concentration of a group of pregnant females who have pre-eclampsia (n=18) is about 85 ng/ml. In contrast, the mean circulating blood concentration of sST2 of a group pregnant females not suffering from pre-eclampsia (n=18) is about 24 ng/ml.

The baseline level or concentration of sST2 may therefore typically be in the range of 10 to 40ng/ml. For example, the baseline level or concentration of sST2 may be about 10ng/ml, 11ng/ml, 12ng/ml, 13ng/ml, 14/ng/ml, 15ng/ml, 20ng/ml, 25ng/ml, 30ng/ml, 35ng/ml or 40ng/ml.

In one embodiment, the sST2 level/concentration in the pregnant female is preferably increased by at least 1.5 fold and typically by at least 2 fold, 3 fold, 4 fold, 5 fold, 6 fold, 7 fold, 8 fold, 9 fold or 10 fold compared to the normal pregnancy level.

According to the present invention, the increase in sST2 level or concentration which is associated with a risk of developing pre-eclampsia is an increase of at least 2 fold, 3 fold, 4 fold, 5 fold, 6 fold, 7 fold, 8 fold, 9 fold or 10 fold relative to the baseline level or concentration.

In the present invention, an increased level or concentration of sST2 associated with a risk of developing pre-eclampsia is typically greater than about 50ng/ml or greater than about 55ng/ml, 60ng/ml, 70ng/ml, 75ng/ml, 80ng/ml, 85ng/ml, 90ng/ml, 95ng/ml, 100ng/ml, 105ng/ml, 110ng/ml, 115ng/ml, 120ng/ml, 125ng/ml or 130ng/ml. According to the invention, the increased concentration of sST2 associated with risk of developing pre-eclampsia is preferably greater than about 50 ng/ml.

According to the present invention, an sST2 level or concentration above the 95% confidence interval for the normal pregnancy level is typically associated with susceptibility to or an increased risk of developing pre-eclampsia.

Also according to the present invention, the level of sST2 may be determined at any stage during pregnancy. The pregnant female being tested may be in the first, second or third trimester of pregnancy. Preferably, the pregnant female being tested is in the late second or early third trimester of pregnancy. The pregnant female may be tested during labour or immediately after delivery. Typically the woman is at a stage of pregnancy from about 20 to 40 weeks gestation, more preferably from about 24 or 26 to 40 weeks gestation. The woman may be at a stage of pregnancy at about 20, 21, 22, 23, 24, 25, 26, 27, 28, 29, 30, 31, 32, 33, 34, 35, 36, 37, 38, 39 or 40 weeks gestation.

According to the present invention, the level of sST2 may be monitored over a period of weeks. Typically, the level of sST2 is monitored (i) once a week; (ii) twice a week, (iii) once every two weeks or (iv) once a month. The level of sST2 does not need to be determined on the same day of each week, or on the same day of each month, although this is encompassed by the method of the invention. For example, it would not be necessary to measure the level of sST2 every Monday, a measurement on a Monday of one week, Wednesday the following week and Tuesday the next week would also be monitoring once a week for the purposes of the invention.

A pregnant female presenting with high blood pressure (rather than pre-eclampsia) or pregnant females known to be at high risk of pre-eclampsia may be tested to give additional information about their risk.

According to the invention, the level of sST2 may be determined multiple times during a pregnancy. Typically, the level of sST2 may be determined at least 1, 2, 3, 4, 5, 6, 7, 8, 9, 10, 16, 20, 25, 30, 35 or 40 times.

Pregnant females may experience risk factors appearing during the course of the pregnancy. Such risk factors include a rising blood pressure on its own, increasing evidence of protein in the urine, on its own, or evidence for slowing or cessation of the unborn baby's growth. According to the present invention, these pregnant females may be monitored up to once or twice a week.

### Detection of sST2

The invention is typically carried out *in vitro* on a sample obtained from the pregnant female. The sample typically comprises a body fluid of the pregnant female. The sample is preferably a blood, plasma, serum or urine sample but may be amniotic fluid.

The sample is typically processed prior to being assayed, for example by centrifugation. The sample may also be typically stored prior to assay, preferably below -80°C.

Any suitable method may be used to determine the level of sST2. Standard methods known in the art may be used to assay the level of sST2. These methods typically involve using an agent for the detection of sST2. The agent typically binds specifically to sST2. The agent may be an antibody specific for sST2. By specific, it will be understood that the agent or antibody binds to sST2 with no significant cross-reactivity to any other molecule, particularly any other protein. For example, an agent or antibody specific for sST2 will show no significant cross-reactivity with human neutrophil elastase. Cross-reactivity may be assessed by any suitable method.

An antibody used in the method of the invention may either be a whole antibody or a fragment thereof which is capable of binding to sST2. The antibody may be monoclonal. Such a whole antibody is typically an antibody which is produced by any suitable method known in the art. For example, polyclonal antibodies may be obtained by immunising a mammal, typically a rabbit or a mouse, with sST2 under suitable conditions and isolating antibody molecules from, for example, the serum of said mammal. Monoclonal antibodies may be obtained by hybridoma or recombinant methods.

Hybridoma methods involve immunising a mammal, typically a rabbit or a mouse, with sST2 under suitable conditions, then harvesting the spleen cells of said mammal and fusing them with myeloma cells. The mixture of fused cells is then diluted and clones are grown from single parent cells. The antibodies secreted by the different clones are then tested for their ability to bind to sST2, and the most productive and stable clone is then grown in culture medium to a high volume. The secreted antibody is collected and purified.

Recombinant methods involve the cloning into phage or yeast of different immunoglobulin gene segments to create libraries of antibodies with slightly different amino acid sequences. Those sequences which give rise to antibodies which bind to sST2 may be selected and the sequences cloned into, for example, a bacterial cell line, for production.

Typically the antibody is a mammalian antibody, such as a primate, human, rodent (e.g. mouse or rat), rabbit, ovine, porcine, equine or camel antibody. The antibody may be a camelid antibody or shark antibody. The antibody may be a nanobody. The antibody can be any class or isotype of antibody, for example IgM, but is preferably IgG.

The fragment of whole antibody that can be used in the method comprises an antigen binding site, e.g. Fab or F(ab)2 fragments. The whole antibody or fragment may be associated with other moieties, such as linkers which may be used to join together 2 or more fragments or antibodies. Such linkers may be chemical linkers or can be present in the form of a fusion protein with the fragment or whole antibody. The linkers may thus be used to join together whole antibodies or fragments which have the same or different binding specificities, e.g. that can bind the same or different polymorphisms. The antibody may be a bispecific antibody which is able to bind to two different antigens, typically any two of the polymorphisms mentioned herein. The antibody may be a 'diabody' formed by joining two variable domains back to back. In the case where the antibodies used in the method are present in any of the above forms which have different antigen binding sites of different specificities then these different specificities are typically to polymorphisms at different positions or on different proteins. In one embodiment the antibody is a chimeric antibody comprising sequence from different natural antibodies, for example a humanised antibody.

Methods to assess sST2 level typically involve contacting a sample with an agent or antibody capable of binding specifically to sST2. Such methods may include dipstick assays and Enzyme-linked Immunosorbant Assay (ELISA). Other immunoassay types may also be used to assess sST2 concentrations. Typically dipsticks comprise one or more antibodies or proteins that specifically bind sST2. If more than one antibody is present, the antibodies preferably have different non-overlapping determinants such that they may bind to sST2 simultaneously.

ELISA is a heterogeneous, solid phase assay that requires the separation of reagents. ELISA is typically carried out using the sandwich technique or the competitive technique. The sandwich technique requires two antibodies. The first specifically binds sST2 and is bound to a solid support. The second antibody is bound to a marker, typically an enzyme conjugate. A substrate for the enzyme is used to quantify the sST2-antibody complex and hence the amount of sST2 in a sample. The antigen competitive inhibition assay also typically requires an sST2-specific antibody bound to a support. An sST2-enzyme conjugate is added to the sample (containing sST2) to be assayed. Competitive inhibition between the sST2-enzyme conjugate and unlabeled sST2 allows quantification of the amount of sST2 in a sample. The solid supports for ELISA reactions preferably contain wells.

The present invention may also employ methods of measuring sST2 that do not comprise antibodies. High Performance Liquid Chromatography (HPLC) separation and fluorescence detection is preferably used as a method of determining the sST2 level. HPLC apparatus and methods as described previously may be used (Tsikas D et al. J Chromatogr B Biomed Sci Appl 1998; 705: 174-6) Separation during HPLC is typically carried out on the basis of size or charge. Prior to HPLC, endogenous amino acids and an internal standard L-homoarginine are typically added to assay samples and these are phase extracted on CBA cartridges (Varian, Harbor City, CA). Amino acids within the samples are preferably derivatized with o-phthalaldehyde (OPA). The accuracy and precision of the assay is preferably determined within quality control samples for all amino acids.

The invention further provides a diagnostic kit that comprises means for measuring the sST2 level in an individual and thereby determining whether or not the pregnant female is at risk of developing pre-eclampsia. The kit typically contains one or more antibodies that specifically bind sST2. For example, the kit may comprise a monoclonal antibody, a polyclonal antibody, a single chain antibody, a chimeric antibody, a CDR-grafted antibody or a humanized antibody. The antibody may be an intact immunoglobulin molecule or a fragment thereof such as a Fab, F(ab')₂ or Fv fragment. If more than one antibody is present, the antibodies preferably have different non-overlapping determinants such that they may bind to sST2 simultaneously.

The kit may additionally comprise means for the measurement of other laboratory or clinical parameters.

The kit may additionally comprise one or more other reagents or instruments which enable any of the embodiments of the method mentioned above to be carried out. Such reagents or instruments include one or more of the following: suitable buffer(s) (aqueous solutions), means to isolate sST2 from sample, means to obtain a sample from the individual (such as a vessel or an instrument comprising a needle) or a support comprising wells on which quantitative reactions can be done. The kit may, optionally, comprise instructions to enable the kit to be used in the method of the invention or details regarding which individuals the method may be carried out upon.

In one embodiment of the invention, additional diagnostic tests may be carried out to confirm the risk of developing pre-eclampsia. These additional tests may be carried out at the same time, before or after the level of sST2 is determined. Two such additional diagnostic tests that may be used are flow-mediated dilatation (FMD) of the brachial artery and or Doppler waveform analysis of the uterine arteries.

sST2 may be measured alongside other markers such as sFlt-1, endoglin and pentraxin 3. sST2 may also be measured alongside independent markers that are known to decrease in pre-eclampsia compared to normal pregnancy, rather than increase. Examples of such an independent marker include PLGF.

Vasodilatation is dependent on the presence of an intact endothelium. Consequently, endothelial dysfunction is reflected by an impaired FMD response. FMD of the brachial artery may be measured in a non-invasive manner using high-resolution ultrasound to detect changes in the diameter of the brachial artery. Ultrasonic apparatus and methods previously described may be used (Savvidou MD et al. Ultrasound Obstet Gynecol 2000; 15: 502-7; Dorup I et al. Am J Physiol 1999; 276: H821-5). The apparatus typically includes a linear array transducer. End-diastolic images of the artery may be stored in digital format. Arterial diameter is typically determined using a semi-automated edge detection algorithm. Baseline vessel diameter is typically calculated as the mean of all the measurements during the first minute of recording. FMD of the brachial artery is defined as the percentage increase in vessel diameter during reactive hyperaemia induced by inflation of a cuff distal to the site of the recording to 300 mmHg for 5 minutes followed by rapid deflation. Flow change (reactive hyperaemia), an index of the flow stimulus for dilation, is typically calculated as [(blood flow 15 sec after cuff deflation-baseline blood flow)/baseline blood flow] X 100%. Endothelium-independent dilatation to sublingual glyceryl trinitrate (GTN) may be used as a control.

FMD of the brachial artery is typically 8.59 ± 2.76% (n=43) in normal pregnant human women. According to the invention, a significant reduction of FMD in addition to an increased sST2 level is indicative of a susceptibility to or a risk of developing pre-eclampsia. The FMD of the brachial artery is preferably reduced by two fold or more preferably by at least two fold.

Doppler analysis is a routine method used to assess the waveform of the uterine arteries. The method is typically used to identify the presence or absence of early diastolic notches in the artery waveform. These notches may be unilateral or bilateral. According to the invention, the presence of a unilateral notch or bilateral notches in addition to an increased sST2 level may be indicative of a susceptibility to pre-eclampsia.

Other assays or genetic tests may be used together with the method of the invention in order to more accurately assess risk of developing pre-eclampsia.

### Example 1

### Methods

### Study population

Forty three pregnant females were identified as suffering from pre-eclampsia. Pre-eclampsia was defined as new hypertension (≥90-mmHg diastolic blood pressure on two occasions) and proteinurea (≥500mg in 24 hours) in the second half of pregnancy compared to diastolic pressures <90mmHg and no detectable proteinuria in the first half of pregnancy. Forty three maternal age, gestational age and parity matched normal pregnant controls were recruited. The birth weights of the babies for both the pre-eclampic and normal pregnant groups were recorded.

### Analysis of circulating blood concentrations of sST2

Blood samples were collected by the attending physician as part of routine patient management at different gestational ages. The concentration of sST2 was determined by ELISA using the Presage assay™ and standard techniques. Briefly, ST2 antigen was provided at a concentration of 400ng/ml. Seven standard concentrations were serially diluted (from 200ng/ml to 0ng/ml). Each patient sample or control sample was diluted 1:50 with a sample dilution buffer. 100µl of standard, control or patient sample was added to the anti sST2 coated plate in duplicate and incubated under agitation for 60 minutes at room temperature. The microtiter plate was washed 5 times and 100µl of Anti-sST2 biotinylated antibody reagent was added to each well. After 60 minutes of incubation at room temperature the plate was washed 5 times. 100µl of Streptavidin-HRP Conjugate Reagent was added to each well and the plate was incubated under agitation for 30 minutes at room temperature and washed 5 times. 100µl of TMB Reagent was added to each well and the plate was incubated under agitation for 20 minutes at room temperature and washed 5 times. Finally, 100µl of Stop solution was added and the plate was read within 15 minutes at an absorbance of 450 nm.

### Localisation of placental ST2

Placental tissue from normal and pre-eclampsic placentas (n=4) was lysed with HEPES lysis buffer and a protease inhibitor. Sample were spun in a microcentrifuge at 13,000g for 5 minutes. Protein concentrations were determined with a bicinchoninice assay kit. 30µg of each sample was loaded onto a SDS-PAGE gel electrophoresis and run for 45 minutes at 150v. Proteins were transferred onto a PVDF membrane by western blotting. Membranes were incubated in blocking buffer (Tris-buffered saline with 5% blotto and 0.1% Tween) for 45 minutes at room temperature. The membrane was incubated with anti ST2 antibody overnight. Reactions were visualized by using anti goat antibody conjugated to horseradish peroxidase in blocking buffer at room temperature for 45 minutes and an enhanced chemiluminescence system.

Soluble ST2 was clearly identified by western blotting (Figure 5) in placentas from both normal and pre-eclamptic patients.

### Statistical analysis

Data were analysed using a paired t-test. A p value of less than 0.05 was considered to be statistically significant.

### Results

### Patient characteristics

There was no difference in the mean age, parity or gestation of the pre-eclampsia and normal pregnant groups. Blood pressure was significantly higher in the pre-eclamptic group (p<0.001) (see Table 1). There was no significant difference in the age of the non-pregnant matches compared to pregnant or pre-eclamptic women (see Table 2).

**Table 1**

| | Age (years) | nulliparity | Gestation (days) | Birthweight (g) | BP (Maximum) |
|---|---|---|---|---|---|
| PE | 31.32 (5.48) | 28/43 | 225.59 (34.34) | 1936.98 (1004.98) | 162.37 (20.60) / 106.80 (12.97) |
| NORMAL | 31.95 (5.33) | 28/43 | 225.15 (25.84) | 3390.34 (390.82) | 121.15 (11.47) / 75.02 (17.27) |

Patient characteristics from normal and pre-eclamptic women (n=43). Values given are means (Standard Deviation). Birthweight and blood pressures (BP) were significantly different between PE and Normal groups (p<0.001), paired t test.

**Table 2**

| | Age (years) | nulliparity | Gestation (days) | Birthweight (g) | Blood Pressure (Maximum) | Proteinurea (mg/l) |
|---|---|---|---|---|---|---|
| PE | 32.06 (4.92) | 11/18 | 228.33 (34.47) | 1936.78 (964.13) | 173 (18.5) / 113 (8.6) | 2075.16 (2214.37) |
| NORMAL | 32.33 (6.70) | 11/18 | 230.61 (35.57) | 3345.22 (649.38) | 130 (7.7) / 79 (7.3) | NAD |
| NON | 31.67 (4.93) | 11/18 | - | - | - | - |

Patient characteristics from non, normal and pre-eclamptic women. Values given are means (Standard Deviation). Birthweight and blood pressures were significantly different between PE and Normal groups (p<0.001), paired t test, n=18.

### Circulating blood levels of sST2

The circulating blood concentration of sST2 was significantly higher in the pre-eclamptic group than in the normal pregnant group (p<0.001) (see Figures 1 and 3, and 2 and 4). sST2 was detected in plasma samples from normal pregnant and pre-eclamptic women. Samples were matched for age (+/- 4 years) and parity (0, 1-3 or 4) and gestational age (+/-13 days). sST2 was detected using the Presage Assay (Critical Diagnostics). Statistical were determined using a paired T test, *** p<0.001. Mean values; normal pregnant = 27.66, pre-eclamptic = 77.70ng/ml. sST2 was detected in plasma samples from non pregnant, normal pregnant and pre-eclamptic women (n=18). Samples were matched for age (+/- 4 years) and parity (0, 1-3 or 4+) in all groups, and for gestational age between normal and pre-eclamptic women (+/-13 days). sST2 was detected using the Presage Assay (Critical Diagnostics). Statistical analysis was performed using a paired t test, ** p<0.01, *** p<0.001. Mean values: non = 18.77, normal pregnant = 31.50, pre-eclamptic =69.70ng/ml.

The largest differences were observed in early-onset pre-eclampsia (21-28 weeks). Levels of sST2 in the early-onset pre-eclamptic group were comparable to those seen after acute myocardial infarction. The circulating blood concentration of sST2 increased in the normal pregnant group with gestational age. In contrast, high levels of sST2 were observed in the pre-eclamptic group from 150 days gestation.

### Localisation of placental ST2

ST2 was identified in placental tissue using western blotting (Figure 5). Soluble ST2 was also identified by Presage assay in the supernatants of perfused placentas from both normal and pre-eclamptic patients (Figure 6). Extracts from normal (n=4) or pre-eclamptic (n=4) placenta were lysed in HEPES lysis solution and 30µg protein were loaded onto a SDS-PAGE gel for electrophoresis. Western blotting was performed for ST2 using ST2 specific antibodies raised in goat (R&D systems) and anti-goat-HRP secondary antibody. sST2 was detected by enhanced chemiluminescence. sST2 is released by the placenta. sST2 was detected in the supernatants from placental perfusions from normal and pre-eclamptic placenta by the Presage Assay (Critical Diagnostics).

### Discussion

There is currently no screening method for pre-eclampsia that is accurate enough for clinical use. This is because to date there has been no biomarker of sufficient specificity and/or sensitivity to develop such a screening method. This is the first study investigating the level of sST2 in the circulating blood of pregnant females and our findings show that sST2 levels are increased in pregnant females suffering from pre-eclampsia. Therefore, sST2 is a useful diagnostic marker for assessing risk of developing pre-eclampsia.

Traditionally, pre-eclampsia has been diagnosed by the presentation of significant hypertension and abnormal proteinuria developing after 20 weeks gestation in a previously normotensive pregnant female. Thus, previous tests for diagnosing pre-eclampsia were only able to diagnose pre-eclampsia after the onset of clinical symptoms.

The method of the invention can be used to assess risk of developing pre-eclampsia. Thus, pregnant females at risk of developing pre-eclampsia can be identified before they present with clinical symptoms. This means that clinical resources can be better directed towards those at risk.

The present study is the first to show that plasma sST2 is increased in pre-eclamptic pregnancies and that the placenta contributes to circulating sST2. Although its role in the disease process is unclear, sST2 is a potential novel biomarker for pre-eclampsia that may be independent of endothelial dysfunction.

In conclusion, this study shows that sST2 is elevated in the circulating blood of pregnant females with pre-eclampsia. Better diagnostic accuracy in assessing risk of developing pre-eclampsia, preferably before the pregnant female presents with symptoms, will allow the clinician to start adequate treatment earlier.

### Additional Studies

### Example 2 - Further Studies

### Materials and Methods

### Subject recruitment and ethics statement

These studies were approved by the Oxfordshire Research Ethics Committee and written consent was obtained from each participant. Three study groups were recruited. To determine changes in sST2 and IL-33 associated with pre-eclampsia, a single blood sample was taken from women with pre-eclampsia (n=20) who were matched for age (+/- 4 years), parity (0, 1-3, 4+), and gestational age (+/- 13 days) to normal pregnant women, and for age and parity to non-pregnant controls (Table 3).

**Table 3 - Clinical characteristics of women with pre-eclampsia, normal pregnant women and non-pregnant women (n=20)**

| | Non-pregnant | Normal Pregnant | Pre-eclamptic | |
|---|---|---|---|---|
| Age (years) | 30.85 (5.38) | 30.7 (5.79) | 29.45 (5.53) | n.s. |
| Nulliparity | 14/20 | 14/20 | 14/20 | n.s. |
| Gestation at sample (days) | | 238.12 (35.63) | 241.45 (32.45) | n.s. |
| Booking systolic BP (mmHg) | | 109.45 (13.04) | 122.50 (14.36) | p<0.05 |
| Booking diastolic BP (mmHg) | | 65.90 (9.96) | 77.15 (14.83) | p<0.05 |
| Max systolic BP (mmHg) | | 124.05 (9.64) | 176.30 (17.54) | p<0.0001 |
| Max diastolic BP (mmHg) | | 75.21 (8.53) | 113.65 (12.42) | P<0.0001 |
| Birthweight (g) | | 3408.45 (402.18) | 2176.25 (1022.97) | p<0.001 |

| | | | | |
|---|---|---|---|---|
| Data are shown as mean (+/- standard deviation) | | | | |

To determine the changes with gestational age in circulating concentrations of sST2 and IL-33, healthy women were recruited to the Oxford Pregnancy Biobank in the first trimester of pregnancy (11-13 weeks gestation), and further samples acquired during the second (20-22 weeks) and third trimesters (30-34 weeks) (characteristics recorded in Table 4).

**Table 4 - Clinical characteristics of women with pre-eclampsia and normal pregnant women (n=15) in the longitudinal study**

| | Non-pregnant | Normal Pregnant | Pre-eclamptic | |
|---|---|---|---|---|
| Age (years) | 28.60 (4.93) | 28.00 (5.50) | 27.93 (5.75) | n.s. |
| Nulliparity | 14/15 | 14/15 | 14/15 | n.s. |
| Gestation at delivery (days) | | 282.00 (6.11) | 250.50 (33.59) | p<0.001 |
| Booking systolic BP (mmHg) | | 109.80 (13.68) | 115.67 (13.64) | n.s. |
| Booking diastolic BP (mmHg) | | 65.53 (8.59) | 70.07 (11.18) | n.s. |
| Max systolic BP (mmHg) | | 125.89 (7.57) | 160.33 (13.22) | p<0.001 |
| Max diastolic BP (mmHg) | | 78.93 (7.25) | 107.13 (8.67) | p<0.001 |
| Birthweight (g) | | 3439.93 (495.84) | 2792.79 (1107.13) | n.s. |

| | | | | |
|---|---|---|---|---|
| Data shown are mean (+/- standard deviation) | | | | |

15 women became pre-eclamptic, these were matched to normal pregnant women. Additional samples were taken from 13/15 of the women who developed pre-eclampsia when diagnosed. 5 of these 15 women had developed pre-eclampsia prior to the third trimester sample being taken. The other 10 women had a third trimester sample taken on average 20 days (range 6-39 days) prior to diagnosis. Blood samples from 15 matched, healthy, non-pregnant women were also taken. Finally, to further study the effect of gestational age on sST2 levels an additional 30 women with pre-eclampsia were matched to normal pregnant women, therefore totalling 50 matched cases of pre-eclampsia (Table 5).

**Table 5 - Clinical characteristics of women with pre-eclampsia and normal pregnant women (n=50)**

| | Normal Pregnant | Pre-eclamptic | |
|---|---|---|---|
| Age (years) | 31.43 (5.58) | 30.80 (5.61) | n.s. |
| Nulliparity | 39/50 | 39/50 | n.s. |
| Gestation at sample (days) | 231.6 (34.87) | 223.1 (34.22) | n.s. |
| Booking systolic BP (mmHg) | 105.7 (11.09) | 120.9 (14.28) | P<0.001 |
| Booking diastolic BP (mmHg) | 64.53 (9.82) | 73.68 (12.59) | P<0.01 |
| Max systolic BP (mmHg) | 125.8 (11.62) | 175.80 (18.13) | P<0.0001 |
| Max diastolic BP (mmHg) | 77.56 (7.12) | 125.30 (33.46) | P<0.0001 |
| Birthweight (g) | 3392.73 (392.76) | 1967.00 (1012.75) | p<0.0001 |

| | | | |
|---|---|---|---|
| Data are shown as mean (+/- standard deviation) | | | |

Pre-eclampsia was defined as the new onset of a diastolic blood pressure (BP) ≥90mmHg on at least two occasions within 24 hours and new onset proteinuria ≥300mg in a 24 hour urine collection, 50mg/mmol protein/creatinine ratio or at least 2+ on dipstick testing on two consecutive measurements. All cases and controls had singleton pregnancies with no known fetal abnormality. Blood samples were collected, plasma and serum were isolated and the samples stored at -80°C until analysis.

There were no significant differences in age, parity or gestation in any of the groups. At the first trimester sample both systolic and diastolic blood pressure was significantly increased in women whose pregnancy was complicated by pre-eclampsia. It is well recognized that increased blood pressure at booking is a risk factor for the disease. Case characteristics are detailed in Tables 1-3.

### Detection of sST2 and IL-33 by ELISA

sST2 was measured in plasma using the Presage® ST2 Assay (Critical Diagnostics, San Diego, USA) according to the manufacturer's instructions. IL-33 was measured in serum or plasma using a human IL-33 ELISA kit with pre-coated plates (BioLegend, San Diego, U.S.A.). We found that the same amount of IL-33 is detected in plasma or serum samples from the same individual (data not shown). For each assay both standards and samples were tested in duplicate.

### Detection of ST2 and IL-33 by immunohistochemistry

Tissue samples from normal (n=3) and pre-eclampsia placentas (n=3) obtained after caesarean section were fixed in 10% formalin and processed into paraffin blocks. Serial sections (5µm) were cut on coated slides and dried. Slides were deparaffinised in Histo-Clear (National Diagnostics, MERCK, Eorulab S.A.), rehydrated in a graded alcohol series, and sodium citrate antigen retrieval performed. Sections were pre-blocked with 10% FCS/PBS for one hour at room temperature. Endogenous peroxidase was quenched with 3% hydrogen peroxide. The sections were incubated overnight at 4°C with 1µg/ml of anti-ST2 antibody (clone 9F8, Critical Diagnostics, San Diego, USA), 1µg/ml of anti-IL-33 antibody clone Nessy-1 (Enzo Life Sciences, New York, USA) or the same concentration of a negative control mouse IgG antibody (Dako, Denmark). A cytokeratin 7 antibody (Dako, Denmark) was used as a positive control to label the syncytiotrophoblast (data not shown). A biotin-free, tyramide signal amplification system was used (Dako, Denmark) for detection, following the manufacturer's instructions. The sections were finally counterstained with haematoxylin. Slides were examined using a Zeiss Axioskop brightfield microscope with a Micropublisher 5MP RT camera.

### Detection of ST2 and IL-33 by immunoblotting

Placental tissue samples (normal (n=9) and pre-eclampsia (n=9)) were lysed using HEPES lysis solution supplemented with Complete Mini protease inhibitor cocktail (Roche, Germany). Placental lysate protein concentrations were determined using a BCA protein assay kit (Pierce, Thermo Scientific, Illinios, USA). Proteins (30µg) were resolved by 10% Bis-Tris Novex SDS-PAGE (Invitrogen, Paisley, UK) and transferred to PVDF membrane. Membranes were incubated for 45 minutes in blocking buffer (PBS with 5% BLOTTO (Santa Cruz, California, USA) and 0.1% Tween) at room temperature. The membranes were then incubated overnight with the appropriate primary antibody at 4°C. Primary antibodies used were goat anti-ST2 (0.5µg/ml) (R&D Systems, Minneapolis, USA), murine anti-IL-33 (0.1µg/ml) clone Nessy-1 (Enzo Life Sciences, New York, USA) or murine anti-β-actin (0.155µg/ml) (Abcam, Massachusetts, USA). Reactions were visualised by incubating the membranes with the appropriate anti-mouse or anti-goat secondary antibody conjugated to horseradish peroxidase (Dako, Denmark) for one hour and detected using an enhanced chemiluminescence system (Pierce, Thermo Scientific, Illinios, USA).

### Placental perfusion model

To determine if IL-33 or sST2 are released from the villous surface into the maternal circulation a modified dual placental perfusion system was used as previously described (Southcombe et al, PloSOne 6:e20245. Placentas were obtained from normal pregnant, healthy women (n=8) or women with pre-eclampsia (n=5) undergoing elective caesarean section, without labor, and were processed immediately. The maternal perfusates were centrifuged to remove cellular debris in a Beckman J6-M centrifuge at 600 g for 10 min at 4°C, and aliquots of the supernatants frozen at -80°C until analysed. sST2 was measured in the perfusate by ELISA as described above, IL-33 was measured using a human IL-33 Duoset ELISA (R&D Systems, Minneapolis, USA).

### Release of sST2 from placenta explants

All placentas were from pregnant, healthy women undergoing elective caesarean section, without labor, and were processed immediately. Freshly delivered placentae (n=3) were first rinsed in ice cold Hanks balanced salt solution and placed into a glove box maintained at 8% O₂. Placental pieces, cut from undamaged lobules that appeared healthy, were rinsed in 8% O₂ equilibrated ice cold explant culture medium (DMEM containing 10% foetal bovine serum (PAA Laboratories GmbH, Austria), 1% antibiotic and antimycotic solution (Sigma Aldrich, UK) and L-glutamine) before being placed into ice cold fresh equilibrated explant culture medium. Placental pieces of approximately 2 mm in diameter were then dissected and distributed equally between Costar Netwell (24 mm diameter, 500 µm mesh) supports in 6-well plates containing 4 ml/well equilibrated explant culture medium (10 explants/well). Placental explants were finally washed again with a medium change before being incubated under conditions 'normoxic' for trophoblast (8% O₂/87 % N₂/5 % CO₂ (Hung et al, Placenta 2008, 29, 565-583) for 24 hours. Explants were also treated with 100ng/ml TNF-α or 100ng/ml IL-1β (Peprotech, NJ, U.S.A.) under normoxic conditions, placed at 1% O₂/94 % N₂/5 % CO₂ to stimulate hypoxic conditions, or placed at 1 % O₂/94 % N₂/5 % CO₂ for 1 hour before switching to 8% O₂/87 % N₂/5 % CO₂ for 23 hours, simulating hypoxia-reoxygenation conditions. After 24hr the explant supernatants were collected and centrifuged at 600g for 10min to remove cell debris. Explants were rinsed in PBS, collected and lysed in HEPES lysis buffer supplemented with Complete Mini protease inhibitor cocktail (Roche, Germany) and protein concentration determined using a BCA Assay (Pierce, Illinois, U.S.A.). The quantity of sST2 released from placenta explants was standardized to the protein concentration of the total explants per condition. Supernatants were collected and stored at -80°C for ELISA. Results were expressed as % sST2 release from explants under varying conditions compared to normoxia (100%). IL-33 was measured in the supernatants by ELISA (R&D Systems).

### Statistical analysis

Gaussian distribution of data was confirmed using a Kolmogorovv-Smimov test. For the longitudinal study, where the data were normally distributed, a repeated measures ANOVA was used with a Bonferroni post hoc test. For non-parametric longitudinal data a Kruskal Wallace test was used with a Dunn's post hoc test. To assess the predictive value of sST2 for pre-eclampsia a ROC curve was generated.Where paired data were shown to be normally distributed, a paired t-test was performed. Non-parametric paired data was analysed using a Wilcoxon matched pairs test. Non matched data comparing non-pregnant to longitudinal data was compared with an unpaired t test. Statistics were generated using Prism and SPSS software. Values of p<0.05 were considered to be statistically significant.

### Results

### There is no significant variation in circulating IL-33 in women with normal or pre-eclamptic pregnancies

IL-33 serum levels were measured in 20 matched trios of women with pre-eclampsia, normal pregnant women and non-pregnant controls (Figure 7A). Despite an increased median value in the pre-eclamptic women there were no significant differences between groups. IL-33 was then measured in serum samples taken in the first, second and third trimester from 15 women who subsequently developed pre-eclampsia and 15 matched normal pregnant controls. An additional sample taken at the time of pre-eclampsia diagnosis was available for 13 of the women. There were no differences in IL-33 levels throughout pregnancy in women who went on to develop pre-eclampsia compared to the normal pregnant controls, or during the active disease (Figure 7B).

### sST2 is increased in the third trimester of normal pregnancy and is significantly elevated in pre-eclampsia prior to the onset of clinical symptoms

Circulating sST2 was next measured in 20 matched trios of women with pre-eclampsia, normal pregnant women and non-pregnant controls. sST2 levels were significantly increased (p<0.001) in women with pre-eclampsia (mean 85.89ng/ml) compared to normal pregnant (mean 25.20ng/ml) and non-pregnant women (mean 20.37ng/ml) (Figure 8A). Plasma sST2 was measured in samples from normal pregnant and pre-eclamptic women throughout each trimester of pregnancy, with an additional sample taken at the time of diagnosis. There was a significant increase in sST2 in the third trimester of normal pregnancy (mean 34.87ng/ml) compared to the first (mean 21.48ng/ml) and second (mean 20.60ng/ml) trimesters (p<0.001) (Figure 8B). Similarly, sST2 levels increased as pregnancy progressed, and levels were significantly higher in the third trimester of pre-eclamptic pregnancies (mean 54.28ng/ml) compared to third trimester samples from normal pregnancies (p<0.001), even before the onset of clinical symptoms. When clinical symptoms were present sST2 levels were further increased (mean 77.82ng/ml). The predictive value of sST2 at the third trimester sample time was assessed by generating a ROC curve, giving an AUC of 0.813, the 95% confidence interval had a lower bound of 0.642 and upper bound of 0.984 (Figure 8C). On average the women (n=10) developed pre-eclampsia 20 days after this sample was taken. No differences between non-pregnant samples and normal pregnant first and second trimester samples were noted, however, as expected, there was a significant increase in the third trimester (p<0.001).

This study was then extended to a larger group of 50 women with pre-eclampsia and matched normal pregnant women (Figure 9). The statistically significant increase in circulating levels of sST2 in the pre-eclampsia group was confirmed (p<0.001) (data not shown). Within the pre-eclamptic patient group the diagnosis was made between 22 weeks and 41 weeks gestation. sST2 levels were therefore compared to gestational age in both groups. In normal pregnancy circulating sST2 levels were strongly correlated with gestational age (r=0.659, p<0.001) (Figure 9). In pre-eclampsia there was a poor correlation with gestational age (r=0.079, p>0.05) as even in early onset pre-eclampsia sST2 levels were high.

### sST2 and IL-33 are expressed by both normal and pre-eclampsia placentas

As circulating sST2 is increased in pregnancy, we speculated that the placenta may be a source. Although significant changes to circulating IL-33 were not detected throughout pregnancy it may be a local mediator that is not detected systemically, therefore we also investigated IL-33 expression. Immunoblotting showed the presence of IL-33 and ST2 in lysates of all normal and pre-eclampsia placental tissues studied (data not shown). Two forms of IL-33 were detected; full length IL-33 was identified in all normal and pre-eclampsia placentas and the shorter cleaved form was identified in several of the placentas. All of the normal and pre-eclamptic placentas expressed ST2. Densitometry was performed to standardise protein expression to actin quantity, no differences between normal and pre-eclamptic placentas were detected (data not shown). Placental sections were then analysed immunocytochemically to determine the cellular localisation of the IL-33 and ST2 (data not shown). Labelling for both molecules was variable. IL-33 localised to the syncytiotrophoblast and the chorionic villi. ST2 localised to some cells in the villous stroma with staining of the syncytiotrophoblast. No differences were seen between normal and pre-eclampsia placentas. No specific staining was seen in IgG controls and the positive control antibody (anti-Cytokeratin-7) strongly stained the syncytiotrophoblast (data not shown).

### The placenta may be the source of some of the sST2 in pregnancy and pre-eclampsia

To determine whether sST2 and IL-33 can be released from the placenta into the maternal circulation, supernatants from the maternal side of placental perfusions (whereby the maternal blood flow across the syncytiotrophoblast is mimicked and maintained *ex vivo)* from normal (n=8) and pre-eclampsia (n=5) placentas were assayed. High levels of sST2 could be detected in all of the perfusates analysed from both normal and pre-eclampsia placentas (Figure 10A). IL-33 was detected by ELISA in the perfusate (Figure 10B) from only one normal placenta. To ensure that the ELISA is able to detect IL-33 in this system, and in the presence of sST2, we preincubated recombinant IL-33 (from the ELISA standards) with culture medium alone or maternal perfusion samples known to contain sST2 for 16 hours prior to ELISA (data not shown). IL-33 could still be detected under each of these conditions, suggesting that neither the media nor sST2 prevent detection by ELISA.

### sST2 secretion from the placenta is increased under conditions mimicking pre-eclampsia

Next we investigated placental release of sST2 and IL-33 changes when treated with pro-inflammatory cytokines, and hypoxia or hypoxia/reoxygenation conditions. Placental explants were treated for 24 hours with TNFα or IL-1β (100ng/ml), sST2 secretion increased by 20.6 and 9.7% respectively (Figure 10C). Explants cultured under hypoxic conditions (1% O₂) released 31.6% less sST2 than explants under normoxic conditions (8% O₂). The greatest increase (37.9%) in sST2 levels was induced by hypoxia/reperfusion conditions (1 hour at 1% O₂ followed by 23 hours at 8% O₂). IL-33 could not be detected in the any of the explant supernatants.

### Discussion

sST2 levels were significantly increased in the third trimester of normal pregnancy and further increased in pre-eclampsia. These increases were detectable prior to the onset of clinical symptoms. Circulating sST2 is increased in a variety of conditions associated with systemic inflammation). Pre-eclampsia, as a systemic inflammatory disease secondary to an oxidatively stressed placenta, can now be included in this list.

The third trimester placenta expresses ST2. Western blotting identified ST2 in all of the placental lysates analysed, no differences between groups were detected. Immunohistochemical staining identified ST2 in the syncytiotrophoblast layer, suggesting it could be secreted into the maternal circulation. Placental secretion of sST2 was first confirmed using a placental perfusion model. Perfusates from all of the normal and pre-eclampsia placentas examined contained sST2, although it was not possible to directly compare the concentrations of sST2 in each of the perfusates - the placentas were from varying gestational ages and the size of placental lobes perfused (and hence the area of syncytiotrophoblast contacted) was variable. sST2 is also produced by alveolar epithelial cells, cardiomyocytes and, to a lesser extent, by brain and small bowel cells, therefore the placenta is unlikely to be the sole source of sST2 in pregnant and pre-eclamptic women. In cardiac conditions associated with ventricular hypertrophy and remodelling increased circulating levels of sST2 are derived from cardiomyocytes activated by mechanical stress or pro-inflammatory stimuli. Strong clinical evidence implicates the endothelium as an additional source, and ST2 is produced by proliferating endothelium *in vitro.* sST2 is also increased in other conditions involving the endothelium such as Dengue Fever or sepsis. Endothelial dysfunction, a well known feature of pre-eclampsia could also contribute to raised levels of sST2 in pre-eclampsia.

Placental explants from normal pregnancies also secreted sST2, and levels were increased in response to pro-inflammatory stimuli (IL-1β or TNF-α) or hypoxia-reoxygenation injury, both of which are features of pre-eclamptic placentas. In other cell types sST2 secretion is stimulated by stress, particularly inflammatory stresses, and has anti-inflammatory actions through its blockade of the danger signal delivered by IL-33. We have previously shown that expression of ST2L is increased on peripheral blood NK and NKT-like cells in pregnancy, which suggested that these might be a target for its ligand IL-33 and contribute to the type 2 bias of normal pregnancy. The production of sST2 may reflect the enhanced 'type 1' inflammatory environment of the disorder compared to normal pregnancy, as sST2 acts as a decoy receptor for IL-33, competing with membrane bound ST2L. Therefore, the very high levels of circulating sST2 seen in pre-eclampsia would be predicted to decrease IL-33 binding to its receptor, thereby contributing to the type 1 bias seen in this disease.

However, here we can find no differences in circulating IL-33 between normal pregnant and non-pregnant women at any stage of gestation. Endothelial cells and epithelial cells constitute major sources *in vivo* and here we show the placenta also expresses IL-33. IL-33 does not have a signal peptide, so must be secreted by one or more non-classical pathways. These are thought to include release after cell necrosis as an alarmin/proinflammatory cytokine with a general role is as an amplifier of innate immune responses. However, IL-33 also acts as an intracellular nuclear factor with transcriptional properties that include repression. To what extent these functions are relevant to the normal pregnancy and the pathogenesis of pre-eclampsia remains to be elucidated.

In summary this study shows for the first time that sST2 increases in the third trimester of normal pregnancy and that sST2 is significantly increased in pre-eclampsia probably secondary to the systemic vascular inflammation of the disorder. In addition we have shown that sST2 is raised in pregnancies destined to be complicated by pre-eclampsia even prior to the onset of clinical symptoms, which proposes its use as a novel biomarker for the disease. We have shown that the origin of at least some sST2 in pregnancy is the placenta and that a stressed placenta, as in pre-eclampsia, secretes more sST2.

## Claims

1. A method of assessing risk of developing pre-eclampsia comprising determining the level of soluble ST2 (sST2) in a sample from a pregnant female and thereby assessing risk of pre-eclampsia, wherein the level of sST2 is determined at any time between 20 and 40 weeks gestation, during labour or immediately after delivery.

2. The method of claim 1, wherein the level of sST2 is determined between 26 and 40 weeks.

3. The method of claim 1 or 2, wherein the level of sST2 is determined one or more times at 26 weeks; 28 weeks; 30 weeks; 32 weeks; 34 weeks; 36 weeks and/or 38 weeks.

4. The method of any one of the preceding claims wherein the level of sST2 is monitored over a period of weeks.

5. The method of claim 3 wherein the level of sST2 is monitored:
(a) once a week;
(b) once every two weeks; or
(c) once a month.

6. The method of any one of the preceding claims wherein the sample is:
(a) a blood sample; or
(b) a urine sample.

7. The method of any one of the preceding claims wherein the level of sST2 is determined by:
(a) an Enzyme-linked Immunosorbant Assay (ELISA); or
(b) a competitive inhibition assay.

8. The method of any one of the preceding claims wherein assessing the risk of developing pre-eclampsia comprises determining whether or not the concentration of sST2 is greater than 50ng/ml in the sample.

9. The method of any one of the preceding claims, wherein the level or concentration of sST2 in the sample is increased by at least 1.5 fold relative to the baseline level or concentration of sST2.

10. The method of any one of the preceding claims wherein the individual is a mammal.

11. The method of claim 8 wherein the mammal is a human.

12. A test kit for use in a method for assessing risk of developing pre-eclampsia, which test kit comprises an agent for the detection of sST2 in a sample from a pregnant female and instructions for carrying out a method of assessing risk of developing pre-eclampsia.

## Patentansprüche

1. Verfahren zum Bewerten des Risikos des Entwickelns von Präeklampsie, welches das Ermitteln des Werts von lösbarem ST2 (sST2) in einer Probe einer Schwangeren und dadurch das Bewerten des Risikos von Präeklampsie umfasst, wobei der Wert von sST2 jederzeit zwischen der 20. und 40. Schwangerschaftswoche, während der Wehen oder unmittelbar nach der Entbindung ermittelt wird.

2. Verfahren nach Anspruch 1, wobei der Wert von sST2 zwischen der 26. und 40. Woche ermittelt wird.

3. Verfahren nach Anspruch 1 oder 2, wobei der Wert von sST2 ein oder mehrere Male in der 26. Woche, 28. Woche, 30. Woche, 32. Woche, 34. Woche, 36. Woche und/oder 38. Woche ermittelt wird.

4. Verfahren nach einem der vorhergehenden Ansprüche, wobei der Wert von sST2 über einen Zeitraum von Wochen überwacht wird.

5. Verfahren nach Anspruch 3, wobei der Wert von sST2:
(a) einmal pro Woche;
(b) einmal alle zwei Wochen; oder
(c) einmal pro Monat
überwacht wird.

6. Verfahren nach einem der vorhergehenden Ansprüche, wobei die Probe:
(a) eine Blutprobe; oder
(b) eine Urinprobe
ist.

7. Verfahren nach einem der vorhergehenden Ansprüche, wobei der Wert von sST2 ermittelt wird durch:
(a) ein Enzyme-linked Immunosorbant Assay (ELISA); oder
(b) ein kompetitives Hemmungs-Assay.

8. Verfahren nach einem der vorhergehenden Ansprüche, wobei das Bewerten des Risikos des Entwickelns von Präeklampsie das Ermitteln umfasst, ob die Konzentration von sST2 in der Probe größer als 50 ng/ml ist oder nicht.

9. Verfahren nach einem der vorhergehenden Ansprüche, wobei der Wert oder die Konzentration von sST2 in der Probe um mindestens das 1,5-fache relativ zum Baseline-Wert oder der Baseline-Konzentration von sST2 erhöht ist.

10. Verfahren nach einem der vorhergehenden Ansprüche, wobei das Individuum ein Säuger ist.

11. Verfahren nach Anspruch 8, wobei der Säuger ein Mensch ist.

12. Test-Set zur Verwendung in einem Verfahren zum Bewerten des Risikos des Entwickelns von Präeklampsie, wobei das Test-Set ein Mittel für die Detektion von sST2 in einer Probe von einer Schwangeren und Anweisungen zum Ausführen eines Verfahrens zum Bewerten des Risikos des Entwickelns von Präeklampsie umfasst.

## Revendications

1. Procédé d'évaluation du risque de développer une pré-éclampsie comprenant la détermination du niveau de ST2 soluble (sST2) dans un échantillon provenant d'une femelle gravide et ainsi l'évaluation du risque de pré-éclampsie, où le niveau de sST2 est déterminé à tout moment entre 20 et 40 semaines de gestation, pendant le travail ou immédiatement après la délivrance.

2. Procédé selon la revendication 1, où le niveau de sST2 est déterminé entre 26 et 40 semaines.

3. Procédé selon la revendication 1 ou 2, où le niveau de sST2 est déterminé une ou plusieurs fois à 26 semaines ; 28 semaines ; 30 semaines ; 32 semaines ; 34 semaines ; 36 semaines et/ou 38 semaines.

4. Procédé selon l'une quelconque des revendications précédentes où le niveau de sST2 est suivi sur une période de semaines.

5. Procédé selon la revendication 3 où le niveau de sST2 est suivi :
(a) une fois par semaine ;
(b) une fois toutes les deux semaines ; ou
(c) une fois par mois.

6. Procédé selon l'une quelconque des revendications précédentes où l'échantillon est :
(a) un échantillon de sang ; ou
(b) un échantillon d'urine.

7. Procédé selon l'une quelconque des revendications précédentes où le niveau de sST2 est déterminé par :
(a) un test à immunosorbant lié à une enzyme (ELISA) ; ou
(b) un test d'inhibition compétitive.

8. Procédé selon l'une quelconque des revendications précédentes, où l'évaluation du risque de développer une pré-éclampsie comprend la détermination de ce que la concentration de sST2 est ou non supérieure à 50 ng/ml dans l'échantillon.

9. Procédé selon l'une quelconque des revendications précédentes, où le niveau ou la concentration de sST2 dans l'échantillon est augmenté d'au moins 1,5 fois par rapport au niveau initial de base ou à la concentration initiale de base de sST2.

10. Procédé selon l'une quelconque des revendications précédentes où l'individu est un mammifère.

11. Procédé selon la revendication 8 où le mammifère est un humain.

12. Kit de test destiné à être utilisé dans un procédé pour évaluer le risque de développer une pré-éclampsie, lequel kit de test comprend un agent pour la détection de sST2 dans un échantillon provenant d'une femelle gravide et des instructions pour mettre en oeuvre un procédé d'évaluation du risque de développer une pré-éclampsie.


### SEQUENCE LISTING

<110> Isis Innovation Limited
<120> Diagnostic Method
<130> N.111372A
<150> GB 1013151.4
   <151> 2010-08-04
<160> 1
<170> PatentIn version 3.5
<210> 1
   <211> 328
   <212> PRT
   <213> Homo sapiens
<400> 1
   Met Gly Phe Trp Ile Leu Ala Ile Leu Thr Ile Leu Met Tyr Ser Thr
   1               5                   10                  15
   Ala Ala Lys Phe Ser Lys Gln Ser Trp Gly Leu Glu Asn Glu Ala Leu
               20                  25                  30
   Ile Val Arg Cys Pro Arg Gln Gly Lys Pro Ser Tyr Thr Val Asp Trp
           35                  40                  45
   Tyr Tyr Ser Gln Thr Asn Lys Ser Ile Pro Thr Gln Glu Arg Asn Arg
       50                  55                  60
   Val Phe Ala Ser Gly Gln Leu Leu Lys Phe Leu Pro Ala Glu Val Ala
   65                  70                  75                  80
   Asp Ser Gly Ile Tyr Thr Cys Ile Val Arg Ser Pro Thr Phe Asn Arg
                   85                  90                  95
   Thr Gly Tyr Ala Asn Val Thr Ile Tyr Lys Lys Gln Ser Asp Cys Asn
               100                 105                 110
   Val Pro Asp Tyr Leu Met Tyr Ser Thr Val Ser Gly Ser Glu Lys Asn
           115                 120                 125
   Ser Lys Ile Tyr Cys Pro Thr Ile Asp Leu Tyr Asn Trp Thr Ala Pro
       130                 135                 140
   Leu Glu Trp Phe Lys Asn Cys Gln Ala Leu Gln Gly Ser Arg Tyr Arg
   145                 150                 155                 160
   Ala His Lys Ser Phe Leu Val Ile Asp Asn Val Met Thr Glu Asp Ala
                   165                 170                 175
   Gly Asp Tyr Thr Cys Lys Phe Ile His Asn Glu Asn Gly Ala Asn Tyr
               180                 185                 190
   Ser Val Thr Ala Thr Arg Ser Phe Thr Val Lys Asp Glu Gln Gly Phe
           195                 200                 205
   Ser Leu Phe Pro Val Ile Gly Ala Pro Ala Gln Asn Glu Ile Lys Glu
       210                 215                 220
   Val Glu Ile Gly Lys Asn Ala Asn Leu Thr Cys Ser Ala Cys Phe Gly
   225                 230                 235                 240
   Lys Gly Thr Gln Phe Leu Ala Ala Val Leu Trp Gln Leu Asn Gly Thr
                   245                 250                 255
   Lys Ile Thr Asp Phe Gly Glu Pro Arg Ile Gln Gln Glu Glu Gly Gln
               260                 265                 270
   Asn Gln Ser Phe Ser Asn Gly Leu Ala Cys Leu Asp Met Val Leu Arg
           275                 280                 285
   Ile Ala Asp Val Lys Glu Glu Asp Leu Leu Leu Gln Tyr Asp Cys Leu
       290                 295                 300
   Ala Leu Asn Leu His Gly Leu Arg Arg His Thr Val Arg Leu Ser Arg
   305                 310                 315                 320
   Lys Asn Pro Ser Lys Glu Cys Phe
                   325